# EUROPEAN PATENT SPECIFICATION

(11) **EP 4 017 597 B1**
(45) Date of publication and mention of the grant of the patent: **28.10.2026**
(21) Application number: 21717984.5
(22) Date of filing: 09.03.2021
(51) Int. Cl.: A61Q 5/00, A61Q 5/02, A61Q 15/00, A61Q 19/00, A61Q 19/10, A61K 8/36, A61K 8/365

(54) **PERSONAL CARE COMPOSITIONS**
KÖRPERPFLEGEZUSAMMENSETZUNGEN
COMPOSITIONS DE SOINS PERSONNELS

(30) Priority: 09.03.2020 US 202062987023 P; 09.03.2020 US 202062987049 P
(43) Date of publication of application: 29.06.2022
(73) Proprietor: Colgate-Palmolive Company, New York, NY 10022 (US)
(72) Inventor: LI, Min, Bridgewater, New Jersey 08807 (US); FAN, Aixing, Bridgewater, New Jersey 08807 (US); BOYD, Thomas, Metuchen, New Jersey 08840 (US); BHARDWAJ, Vinay, Piscataway, New Jersey 08854 (US); NESTA, Jason, Somerset, New Jersey 08873 (US)
(74) Representative: Wichmann, Hendrik
(86) International application number: PCT/US2021/021428
(87) International publication number: WO 2021/183464

(56) References cited:
- WO-A1-2018/022016
- WO-A1-2020/185654
- JP-A- 2004 089 177
- US-A- 5 785 962
- US-A1- 2011 256 082
- DATABASE GNPD [online] MINTEL; 27 November 2012 (2012-11-27), ANONYMOUS: "Super-Mud Clearing Treatment", XP055814782, retrieved from https://www.gnpd.com/sinatra/recordpage/1921209/ Database accession no. 1921209
- DATABASE GNPD [online] MINTEL; 10 November 2017 (2017-11-10), ANONYMOUS: "Promise Keeper Blemish Facial", XP055814783, retrieved from https://www.gnpd.com/sinatra/recordpage/5196555/ Database accession no. 5196555
- DATABASE GNPD [online] MINTEL; 2 April 2019 (2019-04-02), ANONYMOUS: "Supertoner Exfoliating Acid Solution", XP055814786, retrieved from https://www.gnpd.com/sinatra/recordpage/6457009/ Database accession no. 6457009
- DATABASE GNPD [online] MINTEL; 29 April 2019 (2019-04-29), ANONYMOUS: "Superserum 6-Acid Refining Treatment", XP055814788, retrieved from https://www.gnpd.com/sinatra/recordpage/6521601/ Database accession no. 6521601
- DATABASE GNPD [online] MINTEL; 11 June 2014 (2014-06-11), ANONYMOUS: "Clinical Concentrate Radiance Booster", XP055814790, retrieved from https://www.gnpd.com/sinatra/recordpage/2350479/ Database accession no. 2350479
- DATABASE GNPD [online] MINTEL; 29 September 2011 (2011-09-29), ANONYMOUS: "Milk Body Lotion", XP055814794, retrieved from https://www.gnpd.com/sinatra/recordpage/1632704/ Database accession no. 1632704
- DATABASE GNPD [online] MINTEL; 4 November 2011 (2011-11-04), ANONYMOUS: "E.V.E. Essential Vital Elements Serum Source", XP055692005, retrieved from https://www.gnpd.com/sinatra/recordpage/1666406/ Database accession no. 1666406
- DATABASE GNPD [online] MINTEL; 10 October 2006 (2006-10-10), ANONYMOUS: "Micro Exfoliating Oxygenating Gel", XP055814797, retrieved from https://www.gnpd.com/sinatra/recordpage/598917/ Database accession no. 598917
- DATABASE GNPD [online] MINTEL; 22 September 2014 (2014-09-22), ANONYMOUS: "Intimate Wash", XP055814801, retrieved from https://www.gnpd.com/sinatra/recordpage/2677521/ Database accession no. 2677521
- DATABASE GNPD [online] MINTEL; 10 April 2017 (2017-04-10), ANONYMOUS: "Acne On The Spot Serum", XP055814802, retrieved from https://www.gnpd.com/sinatra/recordpage/4688909/ Database accession no. 4688909
- DATABASE GNPD [online] MINTEL; 24 June 2005 (2005-06-24), ANONYMOUS: "Intensity Classic Deo Roll-On", XP055814826, retrieved from https://www.gnpd.com/sinatra/recordpage/371579/ Database accession no. 371579
- DATABASE GNPD [online] MINTEL; 10 April 2018 (2018-04-10), ANONYMOUS: "Shaveless Hair Minimizing Anti-Perspirant Deodorant Roll-On", XP055814852, retrieved from https://www.gnpd.com/sinatra/recordpage/5574907/ Database accession no. 5574907

## Description

### BACKGROUND

Probiotics are well known to have beneficial effects on skin health. Much effort has been put forth to improve the skin microflora balance. In particular, it is desirable to inhibit harmful bacteria, while promoting the growth of beneficial bacteria. However, the ability to provide this dual benefit still remains a challenge.

In addition, skin tends to lose its elasticity and/or firmness as it ages. Current options for maintaining skin elasticity and firmness are sub-optimal.

As such, embodiments of the present invention are designed to provide these, and other, benefits. US 2011/0256082 A1 discloses products and methods for reducing the production of unwanted odors on the human body. The product "Super-Mud Clearing Treatment" by GlamGlow is a mask and spot treatment comprising water, kaolin, magnesium aluminum silicate, sodium hydroxide, eucalyptus globulus, mandelic acid, charcoal, parfum (benzyl benzoate, linalool), glycolic acid, lactic acid, azelaic acid, pyruvic acid, salicylic acid, xanthan gum, aloe barbadensis leaf juice, maltodextrin, iron oxides, peppermint oil, butylene glycol, glycyrrhiza glabra root extract, glycerin, chamomilla recutita flower extract, calendula officinaiis flower extract, cucumis sativus fruit extract, hedera helix extract, symphytum officinale leaf extract, phenoxyethanol, methylparaben, butylparaben, ethylparaben, propylparaben, isobutylparaben, methylchloroisothiazolinone, methylisothiazolinone. The product "Promise Keeper Blemish Facial" by Allies Group is a personal care composition to repair, regenerate and re-train skin and comprising in a cosmetically acceptable carrier, lactic acid, mandelic acid and pyruvic acid. The product "Supertoner Exfoliating Acid Solution" by GlamGlow" is a personal care composition for providing refreshed, toned, refined skin and for removing dead surface cells on the skin, comprising in a cosmetically acceptable carrier, lactic acid, mandelic acid, pyruvic acid, tartaric acid, glycolic acid and citric acid. The product "Superserum 6-Acid Refining Treatment" by GlamGlow is a personal care composition used as a skin-plumping and exfoliating serum, comprising in a cosmetically acceptable carrier, lactic acid, mandelic acid, pyruvic acid, glycolic acid, tartaric acid and sorbic acid. The product "Clinical Concentrate Radiance Booster" by Dr. Dennis Gross Skincare is a personal care composition used as a skin-plumping and exfoliating serum, comprising in a cosmetically acceptable carrier, lactic acid, mandelic acid, pyruvic acid, glycolic acid, tartaric acid and sorbic acid. The product "Milk Body Lotion" by Novartis Consumer Health is a personal care composition in the form of a milk body lotion, comprising in a cosmetically acceptable carrier, lactic acid, sodium pyruvate and citric acid. The product "E.V.E. Essential Vital Elements Serum Source" by Institut Esthederm is a personal care composition to fill wrinkles and fine lines to leave skin looking smooth, comprising in a cosmetically acceptable carrier, lactic acid, sodium citrate, sodium pyruvate, sodium lactate, citric acid, sodium acetate and potassium sorbate. The product "Micro Exfoliating Oxygenating Gel" by Johnson & Johnson is a personal care exfoliating composition to unclog the pores and eliminate excess oil, comprising in a cosmetically acceptable carrier, lactic acid and sodium pyruvate. The product "Intimate Wash" by Coop is a personal care composition used as an intimate wash for feminine hygiene, comprising in a cosmetically acceptable carrier, lactic acid, mandelic acid, potassium sorbate and citric acid. The product "Acne On The Spot Serum" by Skin Design London is a personal care composition for the treatment of skin prone to acne, to control bacteria, to clear dead cells and to prevent blocked pores, comprising in a cosmetically acceptable carrier, lactic acid, mandelic acid and sorbic acid. The product "Intensity Classic Deo Roll-On" by Briseis is a personal care composition, namely a deodorant roll-on, comprising in a cosmetically acceptable carrier, lactic acid and formic acid. The product "Shaveless Hair Minimizing Anti-Perspirant Deodorant" by Personal Collection is a personal care composition, namely an anti-perspirant deodorant roll-on, comprising in a cosmetically acceptable carrier, lactic acid, malic acid and citric acid. JP 2004 089177 A discloses a method of producing lactic acid by hydrolyzing paper manufacture sludge and converting the glucose produced to lactic acid by fermentation using *Lactobacillus rhamnosus.* The culture solution produced/generated through the fermentation process comprises citric acid, succinic acid, lactic acid, formic acid, acetic acid and propionic acid, all falling within the definition of the short chain fatty acids. The culture solution is suitable to be used as a personal care composition. WO 2018/022016 A1 discloses liquid cleansing compositions used as quid hand soap and shower gel used in e.g. hospitals, comprising a cleansing component, and an antibacterial system comprising specified amounts of antibacterial agent and antibacterial enhancing agent such as lactic acid, wherein exemplified compositions comprise, in a cosmetically acceptable carrier, lactic acid (respectively 0,25 wt.%, 1.0 wt.% or 1.80 wt.%), 0.32 wt.% sodium benzoate and 0.23 wt.% sodium salicylate. US 5,785,962 A discloses a shampoo composition providing the hair with improved properties such as combability, volume and lustre, containing at least one anionic, nonionic and (or) zwitterionic (amphoteric) surfactant and a mixture of a) at least 0.1% by wt. lactic acid; b) at least 0.05% by wt. citric acid; and c) at least 0.05% by wt. pyrrolidone carboxylic acid, all percentages being calculated to the total composition.

### BRIEF SUMMARY

The present invention provides personal care compositions comprising: a cosmetically acceptable carrier; a first component comprising a first short chain fatty acid; and a second component comprising a second short chain fatty acid as further defined in the claims.

Further embodiments provide a personal care composition comprising a first short chain fatty acid; a second short chain fatty acid; and a cosmetically acceptable carrier as further defined in the claims.

The compositions can be used in methods of treating a body surface of a mammal (e.g. skin) in need thereof, the method comprising administering to the body surface of the patient, an effective amount of a personal care composition described herein.

### BRIEF DESCRIPTION OF THE DRAWINGS

Figure 1 depicts a comparison of the anti-tyrosinase activity provided by a comparative composition and individual short chain fatty acids.
Figure 2 depicts a comparison of the anti-collagenase activity provided by another comparative composition and individual short chain fatty acids.

### DETAILED DESCRIPTION

For illustrative purposes, the principles of the present invention are described by referencing various exemplary embodiments thereof. Although certain embodiments of the invention are specifically described herein, one of ordinary skill in the art will readily recognize that the same principles are equally applicable to, and can be employed in other apparatuses and methods. Before explaining the disclosed embodiments of the present invention in detail, it is to be understood that the invention is not limited in its application to the details of any particular embodiment shown in the description but is defined in the claims. The terminology used herein is for the purpose of description and not of limitation.

As used herein, the term "SCFA Blend" is intended to refer to any combination of short chain fatty acids described herein.

As used herein and in the appended claims, the singular forms "a", "an", and "the" include plural references unless the context dictates otherwise. The singular form of any class of the ingredients refers not only to one chemical species within that class, but also to a mixture of those chemical species. The terms "a" (or "an"), "one or more" and "at least one" may be used interchangeably herein. The terms "comprising", "including", and "having" may be used interchangeably. The term "include" should be interpreted as "include, but are not limited to". The term "including" should be interpreted as "including, but are not limited to".

As used throughout, ranges are used as shorthand for describing each and every value that is within the range. Any value within the range can be selected as the terminus of the range.

The term "about" when referring to a number means any number within a range of 10% of the number. For example, the phrase "about 2.0 wt.%" refers to a number between and including 1.800 wt.% and 2.200 wt.%.

The abbreviations and symbols as used herein, unless indicated otherwise, take their ordinary meaning. The abbreviation "wt%" means percent by weight with respect to the personal care composition. The symbol "°" refers to a degree, such as a temperature degree or a degree of an angle. The symbols "h", "min", "mL"," nm", "µm" means hour, minute, milliliter, nanometer, and micrometer, respectively. The abbreviation "UV-VIS" as referring to a spectrometer or spectroscopy, means Ultraviolet-Visible. The abbreviation "rpm" means revolutions per minute.

The phrase "MRS agar" refers to De Man, Rogosa and Sharpe agar, which is a selective culture medium designed to favor the growth of *Lactobacillus.* The phrase "TSB medium" refers to tryptic soy broth or trypticase soy broth, which is used in microbiology laboratories as a culture broth to grow aerobic bacteria. The phrase "PBS wash" refers to phosphate-buffered saline wash. The abbreviation "qPCR" is quantitative polymerase chain reaction.

When referring to chemical structures, and names, the symbols "C", "H", and "O" mean carbon, hydrogen, and oxygen, respectively. The symbols "-", "=" and "≡" mean single bond, double bond, and triple bond respectively.

Any member in a list of species that are used to exemplify or define a genus, may be mutually different from, or overlapping with, or a subset of, or equivalent to, or nearly the same as, or identical to, any other member of the list of species. Further, unless explicitly stated, such as when reciting a Markush group, the list of species that define or exemplify the genus is open, and it is given that other species may exist that define or exemplify the genus just as well as, or better than, any other species listed.

For readability purposes, the chemical functional groups are in their adjective form; for each of the adjective, the word "group" is assumed. For example, the adjective "alkyl" without a nouns thereafter, should be read as "an alkyl group".

*Escherichia coli* is a Gram-negative, facultative anaerobic, rod-shaped, coliform bacterium of the genus Escherichia that is commonly found in the lower intestine of warm-blooded organisms (endotherms). Most E. coli strains are harmless, but some serotypes can cause serious food poisoning in their hosts, and are occasionally responsible for product recalls due to food contamination.

*Staphylococcus aureus* is a Gram-positive, round-shaped bacterium that is a member of the Firmicutes, and it is a usual member of the microbiota of the body, frequently found in the upper respiratory tract and on the skin. It is often positive for catalase and nitrate reduction and is a facultative anaerobe that can grow without the need for oxygen. Skin infections are the most common form of *S. aureus* infection. This can manifest in various ways, including small benign boils, folliculitis, impetigo, cellulitis, and more severe, invasive soft-tissue infections.

*S. aureus* is extremely prevalent in persons with atopic dermatitis, more commonly known as eczema. It is mostly found in fertile, active places, including the armpits, hair, and scalp. Large pimples that appear in those areas may exacerbate the infection if lacerated. This can lead to staphylococcal scalded skin syndrome, a severe form of which can be seen in newborns.

*Staphylococcus epidermidis,* a Gram-positive bacterium, is a part of the normal human flora, typically the skin flora, and less commonly the mucosal flora. It is a facultative anaerobic bacteria.

Further tests of adding 0.6 wt. % SCFA Blend into a commercially available body wash and application thereof to a skin tissue, showed the body wash to upregulate hydration related gene and tight junction gene and downregulated inflammation related gene expressions. This revolutionary technology is suitable to body wash, lotion or underarm product to provide skin microbiome benefits.

One of the advantages is that the personal care composition provides for an improved skin microflora balance.

Another advantage is that the application of the personal care composition inhibits the growth of harmful bacteria such as *Escherichia coli, Corynebacterium striatum,* and *Staphylococcus aureus.*

Still another advantage is that the personal care composition promotes the growth of beneficial bacteria, such as *Staphylococcus epidermidis.*

A further advantage of personal care composition is the use of the composition upregulated AQP, FASN, OCLN gene expression.

Additional advantage of personal care composition is the use of the composition downregulated IL8 gene expression.

In some embodiments, the personal care compositions improve skin barrier function and hydration. Without being bound by theory, the present inventors believe that the effects observed with the compositions described herein are the result of increasing filaggrin and occludin expression.

Claim 1 refers to a personal care composition comprising a blend of fatty acids with less than six carbon atoms; and a cosmetically acceptable carrier, wherein the blend of fatty acids comprises: a first fatty acid selected from: lactic acid; a salt thereof; and a combination thereof; and a second fatty acid that is pyruvic acid, or a salt thereof; wherein the weight ratio of the first fatty acid to the second fatty acid is 4 : 1; and wherein the personal care composition is free of acetic acid. In some embodiments, the cosmetically acceptable carrier comprises a surfactant. In some embodiments, the personal care composition further comprises an ingredient known to have antiperspirant or deodorant effect. A personal care composition is any composition that is applied to a person, either neat, or as a mixture.

Personal care compositions include hair care, skin care, sun care, nail care, and oral care compositions. In some embodiments, the personal care composition is applied to the person's skin or scalp. Examples of personal care compositions include an antiperspirant, a deodorant, a body wash, a shower gel, a lotion, a bar soap, a soft soap, a shampoo, a hair conditioner, a sunscreen, a peel (e.g., a chemical peel), and a cosmetic. The personal care composition may be classified and regulated by national or international regulatory agencies as a cosmetic, or as a drug.

In some embodiments, the present invention provides a personal care composition comprising 4-butylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition). In other embodiments, the present invention provides a personal care composition comprising hexylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition). In further embodiments, the present invention provides a personal care composition comprising Silybum Marianum (milk thistle) extract (e.g., from about 0.01 wt.% to about 2 wt.%, optionally about 0.01 wt.%, about 0.05 wt.%, about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, or about 2 wt.%, based on the total weight of the personal care composition). Still further embodiments provide personal care compositions comprising dimethylmethoxy chromanol (e.g., from about 0.01 wt.% to about 1 wt.%, optionally about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.5 wt.%, about 0.75 wt.%, or about 1 wt.%, based on the total weight of the personal care composition). Yet other embodiments provide personal care compositions comprising 4-butylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition); hexylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition); Silybum Marianum (milk thistle) extract (e.g., from about 0.01 wt.% to about 2 wt.%, optionally about 0.01 wt.%, about 0.05 wt.%, about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, or about 2 wt.%, based on the total weight of the personal care composition); and dimethylmethoxy chromanol (e.g., from about 0.01 wt.% to about 1 wt.%, optionally about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.5 wt.%, about 0.75 wt.%, or about 1 wt.%, based on the total weight of the personal care composition). In some embodiments, the personal care composition comprises 4-butylresorcinol; hexylresorcinol; Silybum Marianum (milk thistle) extract; and dimethylmethoxy chromanol, and is in the form of a peel (e.g., a chemical peel).

As used herein, "LMP" refers to a combination of lactic acid, mandelic acid and pyruvic acid.

As used herein, "LMP'" may refer to a combination of lactic acid, mandelic acid and sodium pyruvate.

As used herein, "MP" refers to a combination of mandelic acid and pyruvic acid.

As used herein, "MP'" may refer to a combination of mandelic acid and sodium pyruvate.

As used herein, "LP" refers to a combination of lactic acid and pyruvic acid.

As used herein, "LP'" may refer to a combination of lactic acid and sodium pyruvate.

As used herein, "LM" refers to a combination of lactic acid and mandelic acid.

A deodorant is a composition that is applied to the body of a person to prevent body odor caused by the bacterial breakdown of perspiration. A deodorant may be applied to any part of the body. Under selected embodiments, the deodorant is applicable to armpits and feet.

An antiperspirant is a composition that mitigates body odor as well as prevents sweating by affecting sweat glands. An antiperspirant may be applied to any part of the body, and are generally applied to the underarms.

A body wash is a liquid product used for cleaning the body during showers. A body wash comprises synthetic detergents derived from either petroleum or plant sources. A body wash has a lower pH value than soap, and is typically less drying to the skin than a soap.

Under one embodiment, a shower gel is synonymous with body wash. Under an alternative embodiment, a shower gel has a higher viscosity than body wash and has a more firm consistency. A shower gel may have an ingredient that has a cooling feel.

A lotion is a low-viscosity topical preparation for application to the skin. Lotions are applied to external skin with bare hands, a brush, or a clean cloth. A lotion, such as a hand lotion or a body lotion, provides smoothing, moisturizing, softening and perfuming of the skin. A lotion may be used as a medicine delivery system.

The personal care product of the present invention comprises a blend of short-chain acids as further defined in the claims. Optionally, the blend may further comprise a short chained alcohol or a polyol.

Short-chain acids are acids with less than six carbon atoms. As used herein short-chain acids mean not only hydrocarbyl acids of formula CnH2n+1COOH, wherein n is 0 to 6, but also short-chained acids that are substituted with oxygen containing groups such as alcohols or oxo groups.

Short-chain acids that are hydrocarbyl acids are short-chain fatty acids. Examples of short-chain fatty acids include formic acid, methanoic acid, HCOOH, acetic acid, mandelic acid; ethanoic acid, CH₃COOH, propionic acid, CH₃CH₂COOH, butyric acid, butanoic acid, CH₃CH₂CH₂COOH, isobutyric acid, 2-methylpropanoic acid, (CH₃)₂CHCOOH, valeric acid, pentanoic acid, CH₃CH₂CH₂CH₂COOH, isovaleric acid, 3-methylbutanoic acid, and (CH₃)₂CHCH₂COOH.

Claim 1 provides a personal care composition, wherein the first short chain fatty acid is selected from: lactic acid; a salt thereof; and a combination thereof.

Examples of short-chain acids comprising one carbon include methanoic acid, formic acid, and HCOOH.

Examples of short-chain acids comprising two carbons include ethanoic acid, acetic acid, CH₃COOH, trichloro acetic acid, thio acetic acid, ethanedioic acid, oxalic acid, HOOCCOOH, oxoethanoic acid, glyoxylic acid, formylformic acid, OHCCOOH, 2-hydroxyethanoic acid, glycolic acid, dicarbonous acid, hydroxyacetic acid, and HOCH₂COOH.

Examples of short-chain acids comprising three carbons include propanoic acid, ethanecarboxylic acid, CH₃CH₂COOH, prop-2-enoic acid, acrylic acid, acroleic acid, ethylenecarboxylic acid, propene acid, vinylformic acid, CH₂=CH-COOH, 2-propynoic acid, propiolic acid, acetylene carboxylic acid, propargylic acid, CH≡C-COOH, propanedioic acid, malonic acid, methanedicarboxylic acid, HOOC-CH₂-COOH, 2-hydroxypropanedioic acid, tartronic acid, hydroxymalonic acid, HOOC-CHOH-COOH, oxopropanedioic acid, mesoxalic acid, ketomalonic acid, HOOC-CO-COOH, 2,2-dihydroxypropanedioic acid, dihydroxymalonic acid, mesoxalic acid monohydrate, HOOC-C(OH)₂-COOH, 2-oxopropanoic acid, pyruvic acid, α-ketopropionic acid, acetylformic acid, pyroracemic acid, CH₃-CO-COOH, 2-hydroxypropanoic acid, lactic acid, milk acid, CH₃-CHOH-COOH, 3-hydroxypropanoic acid, hydracrylic acid, CH₂OH-CH₂-COOH, 2,3-dihydroxypropanoic acid, glyceric acid, CH₂OH-CHOH-COOH, 2-oxiranecarboxylic acid, and glycidic acid.

Examples of short-chain acids comprising four carbons include butanoic acid, butyric acid, propanecarboxylic acid, CH₃(CH₂)₂COOH, 2-methylpropanoic acid, isobutyric acid, isobutanoic acid, (CH₃)₂CHCOOH, 2-oxobutanoic acid, alpha-ketobutyric acid, CH₃-CH₂-CO-COOH, 3-oxobutanoic acid, acetoacetic acid, CH₃CO-CH₂-COOH, 4-oxobutanoic acid, succinic semialdehyde, HC(O)-CH₂-CH₂-COOH, (*E*)-butenedioic acid, fumaric acid, trans-1,2-ethylenedicarboxylic acid, 2-butenedioic acid, trans-butenedioic acid, allomaleic acid, boletic acid, donitic acid, lichenic acid, HOOC-CH=CH-COOH, (*Z*)-butenedioic acid, maleic acid, cis-butenedioic acid, maleinic acid, toxilic acid, HOOC-CH=CH-COOH, oxobutanedioic aci, oxaloacetic acid, oxalacetic acid, oxosuccinic acid, HOOC-CH₂-CO-COOH, hydroxybutanedioic acid, malic acid, hydroxybutanedioic acid, HOOC-CH₂-CHOH-COOH, 2,3-dihydroxybutanedioic acid, tartaric acid, 2,3-dihydroxysuccinic acid, threaric acid, racemic acid, uvic acid, paratartaric acid, HOOC(CHOH)₂COOH, (*E*)-but-2-enoic acid, crotonic acid, trans-2-butenoic acid, beta-methylacrylic acid, 3-methylacrylic acid, (*E*)-2-butenoic acid, and CH₃-CH=CH-COOH.

Examples of short-chain acids comprising five carbons include pentanoic acid, valeric acid, valerianic acid, butane-1-carboxylic acid, CH₃(CH₂)₃COOH, 3-methylbutanoic acid, isovaleric acid, (CH₃)₂CH-CH₂-COOH, pentanedioic acid, glutaric acid, propane-1,3-dicarboxylic acid, 1,3-propanedicarboxylic acid, *n*-pyrotartaric acid, HOOC-(CH₂)₃-COOH, 2-oxopentanedioic acid, alpha-ketoglutaric acid, 2-ketoglutaric acid, α-ketoglutaric acid, 2-oxoglutaric acid, oxoglutaric acid, and HOOC-(CH₂)₂-CO-COOH.

Under one embodiment, the SCFA Blend also comprises a short chain alcohol. Examples of a short chain alcohol includes methanol, CH₃OH, ethanol, CH₃CH₂OH, n-propanol, 1-propanol, CH₃-CH₂-CH₂-OH, iso-propanol, 2-propanol, (CH₃)₂CH-OH, *n*-butanol, 1-bunatol, CH₃-CH₂-CH₂-CH₂-OH, *sec*-butanol, 2-butanol, CH₃-CH₂-CHOH-CH₃, *iso*-butanol, (CH₃)₂CH-CH₂-OH, *tert*-butanol, (CH₃)₃C-OH, normal amyl alcohol, pentan-1-ol, CH₃-(CH₂)₄OH, isobutyl carbinol, 3-methylbutan-1-ol, isoamyl alcohol, isopentyl alcohol, (CH₃)₂CH-CH₂-CH₂-OH, active amyl alcohol, 2-methylbutan-1-ol, CH₃-CH₂-C(CH₃)H-CH₂-OH, tertiary butyl carbinol, 2,2-dimethylpropan-1-ol, neopentyl alcohol, (CH₃)₃C-CH₂-OH, 3-pentanol, pentan-3-ol, (CH₃-CH₂)CH-OH, methylpropyl carbinol, pentan-2-ol, CH₃-CH₂-CH₂-CH(OH)-CH₃, methyl isopropyl carbinol, 3-methylbutan-2-ol, (CH₃)₂CH-C(OH)-CH₃, dimethyl ethyl carbinol, 2-methylbutan-2-ol, tertiary amyl alcohol, CH₃-CH₂-C(CH₃)₂-OH.

Under one embodiment, the SCFA Blend also comprises a short chain polyol. A polyol is an organic compound containing multiple hydroxyl groups. Examples of polyols include a diol, a triol, and a tetrol. An example of a triol is a glycerol.

Lactic acid is a carboxylic acid with the formula CH3-CH(OH)-COOH. Under one embodiment, the lactic acid is L-(+)-lactic acid or (S)-lactic acid. Under another embodiment the lactic acid is D-(-)-lactic acid of (R)-lactic acid. Under yet another embodiment the lactic acid is a mixture of the two stereoisomers.

The personal care composition can comprise about 0.1 wt% to about 2.0 wt% of the SCFA Blend. Under one embodiment, the personal care composition comprises about 0.1 wt% to about 0.3 wt% of the SCFA Blend. Under one embodiment, the personal care composition comprises about 0.1 wt% to about 0.5 wt% of the SCFA Blend. Under one embodiment, the personal care composition comprises about 0.1 wt% to about 0.7 wt% of the SCFA Blend. Under one embodiment, the personal care composition comprises about 0.1 wt% to about 1.1 wt% of the SCFA Blend. Under one embodiment, the personal care composition comprises about 0.1 wt% to about 1.3 wt% of the SCFA Blend. Under one embodiment, the personal care composition comprises about 0.1 wt% to about 1.5 wt% of the SCFA Blend. Under one embodiment, the personal care composition comprises about 0.1 wt% to about 1.7 wt% of the SCFA Blend.

Under one embodiment, the personal care composition comprises about 0.3 wt% to about 0.5 wt% of the SCFA Blend. Under one embodiment, the personal care composition comprises about 0.3 wt% to about 0.7 wt% of the SCFA Blend. Under one embodiment, the personal care composition comprises about 0.3 wt% to about 1.1 wt% of the SCFA Blend. Under one embodiment, the personal care composition comprises about 0.3 wt% to about 1.3 wt% of the SCFA Blend. Under one embodiment, the personal care composition comprises about 0.3 wt% to about 1.5 wt% of the SCFA Blend. Under one embodiment, the personal care composition comprises about 0.3 wt% to about 1.7 wt% of the SCFA Blend. Under one embodiment, the personal care composition comprises about 0.3 wt% to about 2.0 wt% of the SCFA Blend.

Under one embodiment, the personal care composition comprises about 0.5 wt% to about 0.7 wt% of the SCFA Blend. Under one embodiment, the personal care composition comprises about 0.5 wt% to about 1.1 wt% of the SCFA Blend. Under one embodiment, the personal care composition comprises about 0.5 wt% to about 1.3 wt% of the SCFA Blend. Under one embodiment, the personal care composition comprises about 0.5 wt% to about 1.5 wt% of the SCFA Blend. Under one embodiment, the personal care composition comprises about 0.5 wt% to about 1.7 wt% of the SCFA Blend. Under one embodiment, the personal care composition comprises about 0.5 wt% to about 2.0 wt% of the SCFA Blend.

Under one embodiment, the personal care composition comprises about 0.7 wt% to about 1.1 wt% of the SCFA Blend. Under one embodiment, the personal care composition comprises about 0.7 wt% to about 1.3 wt% of the SCFA Blend. Under one embodiment, the personal care composition comprises about 0.7 wt% to about 1.5 wt% of the SCFA Blend. Under one embodiment, the personal care composition comprises about 0.7 wt% to about 1.7 wt% of the SCFA Blend. Under one embodiment, the personal care composition comprises about 0.7 wt% to about 2.0 wt% of the SCFA Blend.

Under one embodiment, the personal care composition comprises about 0.9 wt% to about 1.1 wt% of the SCFA Blend. Under one embodiment, the personal care composition comprises about 0.9 wt% to about 1.3 wt% of the SCFA Blend. Under one embodiment, the personal care composition comprises about 0.9 wt% to about 1.5 wt% of the SCFA Blend. Under one embodiment, the personal care composition comprises about 0.9 wt% to about 1.7 wt% of the SCFA Blend. Under one embodiment, the personal care composition comprises about 0.9 wt% to about 2.0 wt% of the SCFA Blend.

Under one embodiment, the personal care composition comprises about 1.1 wt% to about 1.3 wt% of the SCFA Blend. Under one embodiment, the personal care composition comprises about 1.1 wt% to about 1.5 wt% of the SCFA Blend. Under one embodiment, the personal care composition comprises about 1.1 wt% to about 1.7 wt% of the SCFA Blend. Under one embodiment, the personal care composition comprises about 1.1 wt% to about 2.0 wt% of the SCFA Blend.

Under one embodiment, the personal care composition comprises about 1.3 wt% to about 1.5 wt% of the SCFA Blend. Under one embodiment, the personal care composition comprises about 1.3 wt% to about 1.7 wt% of the SCFA Blend. Under one embodiment, the personal care composition comprises about 1.3 wt% to about 2.0 wt% of the SCFA Blend. Under one embodiment, the personal care composition comprises about 1.5 wt% to about 1.7 wt% of the SCFA Blend. Under one embodiment, the personal care composition comprises about 1.5 wt% to about 2.0 wt% of the SCFA Blend. Under one embodiment, the personal care composition comprises about 1.7 wt% to about 2.0 wt% of the SCFA Blend.

The personal care composition of the present invention comprises a blend of short chain fatty acids, wherein the short chain fatty acids are lactic acid and pyruvic acid.

**In** some embodiments, the complexes described herein may comprise a SCFA Blend alone, or a SCFA Blend in combination with other ingredients.

**In** some embodiments, the personal care composition includes a surfactant. The surfactant can be any anionic, nonionic, amphoteric, or zwitterionic surfactant, or combinations thereof. The amount of surfactant in the composition is at least 1 weight %. In other embodiments, the amount is 1 to 20 weight %, or at least 2, 3, 4, 5, 6, 7, 8, 9, or 10 weight %.

Under various embodiments, surfactants make up 10, 20, 30, 40, or 50 percent of the personal care composition. Under an embodiment, multiple surfactants are used to achieve desired product qualities. A primary surfactant provides good foaming ability and cleaning effectiveness, while a secondary surfactant adds qualities of mildness to prevent irritation or over-drying of the skin. To prevent ingredients from separating, emulsifiers such as diethanolamine may be added. Additional ingredients include conditioning agents that moisturize the skin during and after product use. Ingredients, like scent in the form of essential oils or fragrance oils, and colorant in the form of water soluble dyes may also be used.

A variety of anionic surfactants can be utilized in the personal care composition including, for example, long chain alkyl (C₆-C₂₂) materials such as long chain alkyl sulfates, long chain alkyl sulfonates, long chain alkyl phosphates, long chain alkyl ether sulfates, long chain alkyl alpha olefin sulfonates, long chain alkyl taurates, long chain alkyl isethionates (SCI), long chain alkyl glyceryl ether sulfonates (AGES), sulfosuccinates and the like. These anionic surfactants can be alkoxylated, for example, ethoxylated, although alkoxylation is not required. These surfactants are typically highly water soluble as their sodium, potassium, alkyl and ammonium or alkanol ammonium containing salt form and can provide high foaming cleansing power. Other equivalent anionic surfactants may be used. In one embodiment, the anionic surfactant comprises sodium laureth sulfate, sodium pareth sulfate, and combinations thereof. Anionic surfactants can be included in any desired amount. In one embodiment, anionic surfactants are present in the composition in an amount of 0 to about 15% by weight. In one embodiment, anionic surfactants are present in an amount of about 6 to about 8% by weight.

Amphoteric surfactants may also be included in the composition. These surfactants are typically characterized by a combination of high surfactant activity, lather forming and mildness. Amphoteric surfactants include, but are not limited to derivatives of aliphatic secondary and tertiary amines in which the aliphatic radical can be straight chain or branched and wherein one of the aliphatic substituents contains about 8 to about 18 carbon atoms and one contains an anionic water solubilizing group, *e.g.,* carboxy, sulfonate, sulfate, phosphate, or phosphonate. Examples of such compounds include sodium 3-dodecyaminopropionate, sodium 3-dodecylaminopropane sulfonate, N-alkyl taurines and N-higher alkyl aspartic acids. Other equivalent amphoteric surfactants may be used. Examples of amphoteric surfactants include, but are not limited to, a range of betaines including, for example, high alkyl betaines, such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, sulfobetaines such as coco dimethyl sulfopropyl betaine, stearyl dimethyl sulfopropyl betaine, amido betaines, amidosulfobetaines and the like. Betaines having a long chain alkyl group, particularly coco, may be particularly useful as are those that include an amido groups such as the cocamidopropyl and cocoamidoethyl betaines. Amphoteric surfactants can be included in any desired amount. In one embodiment, amphoteric surfactants are present in the composition in an amount of 0 to about 15% by weight. In one embodiment, the amphoteric surfactants are present in the composition in an amount of about 4 to about 6% by weight.

Examples of nonionic surfactants include polysorbate 20, long chain alkyl glucosides having C₈-C₂₂ alkyl groups; coconut fatty acid monoethanolamides such as cocamide MEA; coconut fatty acid diethanolamides, fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (for example the PLURONIC^{™} block copolymers commercially available from BASF); fatty acid alkylolamides, (fatty acid amide polyethylene glycols); N-alkyl-, N-alkoxypolyhydroxy fatty acid amides; sucrose esters; sorbitol esters; polyglycol ethers; and combinations thereof. Nonionic surfactants can be included in any desired amount. In one embodiment, nonionic surfactants are present in the composition in an amount of 0 to about 3% by weight. In one embodiment, nonionic surfactants are present in the composition in an amount of about 0.5 to about 1.5% by weight.

Cationic surfactants can also be included in the composition. Examples of cationic surfactants include any quaternium or polyquaternium compound. Cationic surfactants can be included at any desired level. In one embodiment, cationic surfactants are present in the composition in an amount of 0 to about 2% by weight. In one embodiment, cationic surfactants are present in the composition in an amount of about 0.1 to about 0.3% by weight.

Additional ingredients may be present in the personal care composition. These include water and ingredients to thicken, preserve, emulsify, add fragrance, and color.

Skin compatible oils can be included in the composition. Skin compatible oils include a range of liquid hydrocarbons, for example, linear and branched oils such as liquid paraffin, squalene, squalane, mineral oil, low viscosity synthetic hydrocarbons such as polyalphaolefins, commercially available from ExxonMobil under the trade name PURESYN PAO and polybutene under the trade name PANALANE^{™} or INDOPOL^{™}. Light (low viscosity) highly branched hydrocarbon oils may also be suitable in some instances. Other useful skin compatible oils may be silicone based, for example, linear and cyclic polydimethyl siloxane, organo functional silicones (alkyl and alkyl aryl), and amino silicones.

In other embodiments, the composition may include any of following materials in any desired amount to achieve a desired effect in the composition (amounts that can be used in some embodiments are provided): one or more alkaline salts, for example, sodium chloride, sodium sulfate, sodium carbonate, sodium bicarbonate and/or their equivalents (0 to 5% by weight); foaming agents, for example decyl glucoside, and/or their equivalents (0 to 3% by weight); glyceryl esters and derivatives, for example glycol distearate, and/or their equivalents (0 to 3%; by weight); sequestrants, for example, tetrasodium EDTA, and/or their equivalents (0 to 2% by weight); biocides, for example, Triclosan (2,4,4'-trichloro-2'-hydroxydiphenyl ether), DMDM hydantoin, formaldehyde and/or imidazolidinyl urea, and/or their equivalents (0 to 2% by weight); organic acids, for example, citric acid and/or formic acid and/or their equivalents (0 to 2% by weight); viscosity modifiers (0 to 2% by weight); fragrances and/or perfumes (0 to 5% by weight); preservatives, for example, phenoxyethanol, formaldehyde solution, parabens, pentanediol or sorbic acid (0 to 2% by weight); pearlizing agents, for example, glycol distearic esters, such as ethylene glycol distearate, but also fatty acid monoglycol esters (0 to 3% by weight); stabilizers, for example, metal salts of fatty acids, such as e.g. magnesium stearate, aluminum stearate and/or zinc stearate (0 to 2% by a weight); and dyes and pigments that are approved and suitable for cosmetic purposes.

Water may be included in the composition. Water can be included in an amount of 0 to about 90% by weight. In one embodiment, water is present at about 50% to about 90% by weight.

In one embodiment, a moisturizing body wash composition also utilizes, as a thickening agent, a blend of PEG-150 distearate and PPG-2 hydroxyethyl cocamide for countering a decrease in viscosity associated with the concentrations of moisturizing agents utilized in some embodiments of the moisturizing body wash composition. This blended thickening agent allows the composition to achieve viscosities beyond those that could be achieved with conventional thickening agents, for example sodium chloride alone, and is able to achieve suitable viscosities at relatively low concentrations. The relatively low concentrations used to achieve the desired viscosities are also advantageous with respect to manufacturing processes that may be employed to manufacture the moisturizing body wash composition, thereby reducing the need for larger equipment or modifications and the capital expenditure associated with manufacturing the moisturizing body wash composition if other thickening agents were used. The PEG-150 distearate and the PPG-2 hydroxyethyl cocamide can be present in any amount to achieve a desired viscosity. In one embodiment, the amount of PEG-150 distearate in the composition is 0 to about 2% by weight. In one embodiment, the amount of PPG-2 hydroxyethyl cocamide in the composition is 0 to about 2% by weight. In one embodiment, the weight ratio of the PEG-150 distearate to the PPG-2 hydroxyethyl cocamide can be about 3:1 to about 1:3. In one embodiment the PEG-150 distearate and the PPG-2 hydroxyethyl cocamide are each present at 0.0225% by weight. The PEG-150 distearate and the PPG-2 hydroxyethyl cocamide are available as a mixture from Uniqema under the trade name PROMIDIUM^{™} LTS.

Microbeads may also be added to the personal care composition. Microbeads are microspheres that may added to a variety of cosmetic products for their exfoliating qualities.

Personal care composition may also contain the ingredient menthol, which gives a cooling and stimulating sensation on the skin, and some men's shower gels are also designed specifically for use on hair and body. Shower gels contain milder surfactant bases than shampoos, and some also contain gentle conditioning agents in the formula. This means that shower gels can also double as an effective and perfectly acceptable substitute to shampoo, even if they are not labelled as a hair and body wash. Washing hair with shower gel should give approximately the same result as using a moisturizing shampoo

The personal care composition can also be used in a method of treating a skin of a patient in need thereof, the method comprising administering to the skin the above described composition, effective to promote the growth of beneficial bacteria and inhibit the growth of harmful bacteria, wherein the beneficial bacterium is selected from the group consisting of S. epidermidis, and harmful bacteria is selected from the group consisting of *E. coli, C. striatum,* and *S. aureus.*

The composition is spread on the skin neat, or it may be admixed with water. The spreading of the personal care composition may be done by hand, or it may be done by a instrument such as a glove or a piece of cloth.

The method of applying the personal care composition leaves behind a film. The thickness of the film depends on other parts of the formulation, but for a lotion, the thickness will be comparable to the thicknesses of other lotions, body washes, or deodorants. For those embodiments wherein the personal care composition is a lotion, the thickness is about 50 µm.

The personal care composition can also be used in a method of treating the skin of a patient, wherein the amount of harmful bacteria is reduced by at least about 10%, as measured after 48 hours on a 50 µm thick layer of personal care composition comprising 0.6 wt.% SCFA Blend, compared to the personal care composition not comprising the SCFA Blend. In some embodiments, it is disclosed a method of treating the skin of a patient, wherein the amount of harmful bacteria is reduced by about 10%, as measured after 48 hours on a 50 µm thick layer of personal care composition comprising 0.6 wt.% SCFA Blend, compared to the personal care composition not comprising the SCFA Blend.

The personal care composition can also be used in a method of treating the skin of a patient, wherein the amount of beneficial bacteria is increased by at least about 10%, as measured after 48 hours on a 50 µm thick layer of personal care composition comprising 0.6 wt.% SCFA Blend, compared to the personal care composition not comprising the SCFA Blend. In some embodiments, it is disclosed a method of treating the skin of a patient, wherein the amount of beneficial bacteria is increased by about 10%, as measured after 48 hours on a 50 µm thick layer of personal care composition comprising 0.6 wt.% SCFA Blend, compared to the personal care composition not comprising the SCFA Blend.

Some embodiments of the present invention provide personal care compositions comprising a cosmetically acceptable carrier; and a blend of two short chain fatty acids as defined in the claims. The blend of two short chain fatty acids comprises a first short chain fatty acid; and a second short chain fatty acid as further defined in the claims. In some embodiments, the personal care composition is substantially free of a third short chain fatty acid. The personal care composition is free of acetic acid. In some embodiments, the personal care composition does not contain a third short chain fatty acid.

As used herein, the term "substantially free" is intended to mean a negligible amount, for example, less than about 3 wt.% of the composition, and preferably less than 1 wt.% of the composition.

The weight ratio of the first short chain fatty acid (e.g. lactic acid) to the second short chain fatty acid (pyruvic acid or a salt thereof) is 4 : 1 as defined in the claims.

The personal care composition can be used in a method of treating, preventing or ameliorating a symptom associated with a disease, disorder or condition of the skin, comprising administering to the skin of a subject in need thereof, an effective amount of a personal care composition comprising: a cosmetically acceptable carrier; and a blend of two short chain fatty acids.

The personal care composition can be used in a method of increasing the firmness and/or elasticity of the skin of a patient in need thereof, comprising administering to the skin of said patient, an effective amount of a personal care composition comprising: a cosmetically acceptable carrier; and a blend of two short chain fatty acids.

The personal care composition can be used in a method for inhibiting: tyrosinase; collagenase; and/or elastase, in a subject in need thereof, comprising administering to the skin of said subject, an effective amount of a personal care composition comprising: a cosmetically acceptable carrier; and a blend of two short chain fatty acids.

For avoidance of doubt, salts of any one of the short chain fatty acids described herein, would be suitable for use in the personal care compositions of the present invention.

The following examples further describe and demonstrate illustrative embodiments within the scope of the present invention. Various modifications of the invention in addition to those shown and described herein should be apparent to those skilled in the art and are intended to fall within the appended claims.

### EXAMPLES

Examples which do not fall under the scope of the claims are comparative examples.

### Example 1

The anti-tyrosinase activity of an exemplary composition of the present invention was compared to the anti-tyrosinase activity of individual short chain fatty acids. Assays were performed using a tyrosinase inhibitor screening kit from Sigma Aldrich; and following the manufacturer's protocol. The results of these experiments are described in Table 1 (below).

**Table 1**

| **Sample** | **Wt.%** | **% Tyrosinase Inhibition** |
|---|---|---|
| Lactic Acid (L) | 0.312 | 52.72 |
| Pyruvic Acid (P) | 0.078 | 29.29 |
| L + P | 0.312 (L) + 0.078 (P) | 92.89 |

As demonstrated by the data described in Table 1 (above), an exemplary composition of the present invention provides synergistic inhibition of tyrosinase.

### Example 2

The anti-collagenase activity of an exemplary composition of the present invention was compared to the anti-collagenase activity of individual short chain fatty acids. Assays were performed using a collagenase inhibitor screening kit from Sigma Aldrich; and following the manufacturer's protocol. The results of these experiments are described in Table 2 (below).

**Table 2**

| **Sample** | **Wt.%** | **% Collagenase Inhibition** |
|---|---|---|
| Lactic Acid (L) | 1.25 | 40.32 |
| Pyruvic Acid (P) | 0.312 | 8.79 |
| L + P | 1.25 (L) + 0.312 (P) | 78.92 |

As demonstrated by the data described in Table 2 (above), an exemplary composition of the present invention provides synergistic inhibition of collagenase.

### Example 3

The anti-elastase activity of an exemplary composition of the present invention was compared to the anti-elastase activity of individual short chain fatty acids. Assays were performed using an elastase inhibitor screening kit from Sigma Aldrich; and following the manufacturer's protocol. The results of these experiments are described in Table 3 (below).

**Table 3**

| **Sample** | **Wt.%** | **% Elastase Inhibition** |
|---|---|---|
| Lactic Acid (L) | 0.625 | 44.89 |
| Pyruvic Acid (P) | 0.156 | 20.21 |
| L + P | 0.625 (L) + 0.156 (P) | 71.10 |

As demonstrated by the data described in Table 3 (above), an exemplary composition of the present invention provides synergistic inhibition of elastase.

### Example 4

The anti-tyrosinase activity of a comparative composition was compared to the anti-tyrosinase activity of individual short chain fatty acids. Assays were performed using a tyrosinase inhibitor screening kit from Sigma Aldrich (MAK 257 Kit); and following the manufacturer's protocol. The results of these experiments are described in Table 4 (below).

**Table 4**

| **Sample** | **Wt.%** | **% Tyrosinase Inhibition** |
|---|---|---|
| Lactic Acid (L) | 0.312 | 1.23 |
| Mandelic Acid (M) | 0.156 | 0.06 |
| L + M | 0.312 (L) + 0.156 (M) | 23.94 |

As demonstrated by the data described in Table 4 (above), a comparative composition provides synergistic inhibition of tyrosinase.

### Example 5

The anti-collagenase activity of a comparative composition was compared to the anti-collagenase activity of individual short chain fatty acids. Assays were performed using a collagenase inhibitor screening kit from Sigma Aldrich (MAK 293 Kit); and following the manufacturer's protocol. The results of these experiments are described in Table 5 (below).

**Table 5**

| **Sample** | **Wt.%** | **% Collagenase Inhibition** |
|---|---|---|
| Lactic Acid (L) | 1.25 | 12.71 |
| Mandelic Acid (P) | 0.625 | 8.57 |
| L + M | 1.25 (L) + 0.625 (M) | 43.16 |

As demonstrated by the data described in Table 5 (above), a comparative composition provides synergistic inhibition of collagenase.

### Example 6

A study of tyrosinase inhibition is conducted (e.g., Sigma Aldrich MAK 257 Kit) to investigate the efficacy of a comparative composition (0.312% mandelic acid and 0.156% pyruvic acid) and individual short chain fatty acids at the same concentrations.

**Table 6**

| **Sample** | **Wt.%** | **% Tyrosinase Inhibition** |
|---|---|---|
| Mandelic Acid (M) | 0.312 | 1.17 |
| Pyruvic Acid (P) | 0.156 | 26.62 |
| M + P | 0.312 (M) + 0.156 (P) | 92.51 |

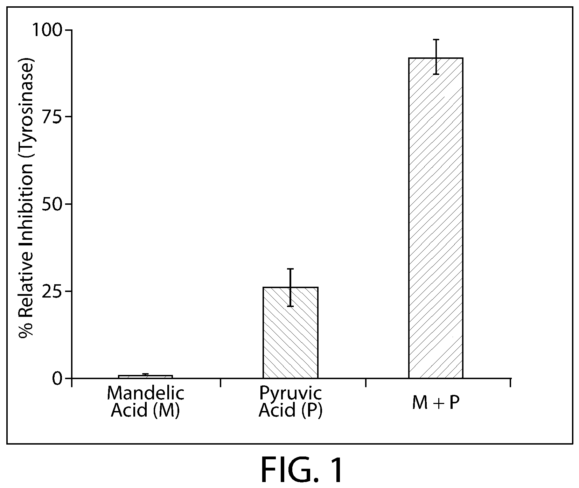

As described by the data reported in Figure 1 a strong synergy between mandelic acid and pyruvic acid was confirmed. The raw data that generated the bar graph depicted in Figure 1, is described above in Table 6.

### Example 7

A study of collagenase inhibition is conducted (e.g., Sigma Aldrich MAK 293 Kit) to investigate the was conducted to investigate the efficacy of a comparative composition (1.25% mandelic acid and 0.625% pyruvic acid) and individual short chain fatty acids, at the same concentrations.

**Table 7**

| **Sample** | **Wt.%** | **% Collagenase Inhibition** |
|---|---|---|
| Mandelic Acid (M) | 1.25 | 11.26 |
| Pyruvic Acid (P) | 0.625 | 9.58 |
| M + P | 1.25 (M) + 0.625 (P) | 27.87 |

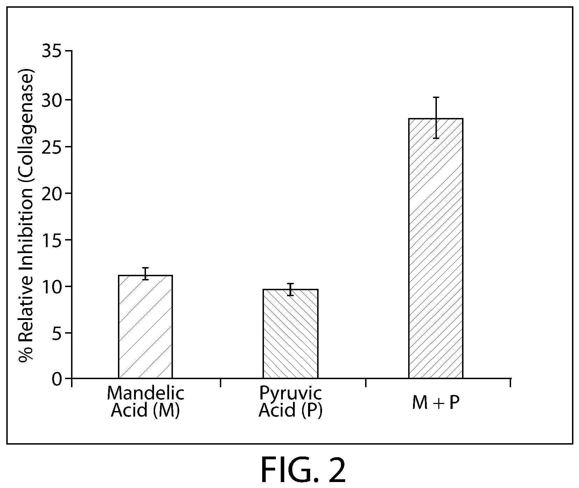

As described by the data reported in Figure 2 a strong synergy between mandelic acid and pyruvic acid was confirmed. The raw data that generated the bar graph depicted in Figure 2, is described above in Table 7.

### Example 8

Peels containing 16 wt.% lactic acid (L); 8 wt.% mandelic acid (M); and 4 wt.% of either pyruvic acid (P) or sodium pyruvate (P') [weight ratio of 4 : 2 : 1] were formulated, to compare the efficacy of pyruvic acid and sodium pyruvate, and to also evaluate the effect of pH on the activity of a peel.

Peels were diluted 1000X in buffer to prepare 0.1% working stock. The working stock was serially diluted 2X to test the peels at five doses (0.1 wt.%, 0.05 wt.%, 0.025 wt.%, 0.0125 wt.% and 0.00625 wt.%). The pH of all test samples and dilutions was measured before and after tyrosinase assay to exclude any effect of acid on the enzyme activity. The assay was performed using a commercial kit following manufacturer's protocol (e.g., MAK 257 Kit from Sigma Aldrich). LMP peels containing various concentrations of pyruvic acid at pH 2.5 ("R1 Peels"); LMP peels which were identically formulated to the R1 Peels, but with pH buffered to 3.6 ("R2 Peels"); and LMP' peels which were identically formulated to the R2 Peels, other than the replacement of pyruvic acid with sodium pyruvate ("R3 Peels"), were all evaluated. The data generated from these evaluations are described in Table 8 below.

**Table 8**

| **Peel** | **Wt.%** | **% Tyrosinase Inhibition** |
|---|---|---|
| **R1** | | |
| | 0.1 | 98.08221 |
| | 0.05 | 92.88508 |
| | 0.025 | 62.24947 |
| | 0.0125 | 26.08278 |
| | 0.00625 | 11.30976 |
| **R2** | | |
| | 0.1 | 99.56708 |
| | 0.05 | 99.89873 |
| | 0.025 | 98.54957 |
| | 0.0125 | 98.62214 |
| | 0.00625 | 85.9364 |
| **R3** | | |
| | 0.1 | 99.94533 |
| | 0.05 | 99.18071 |
| | 0.025 | 99.85245 |
| | 0.0125 | 97.90398 |
| | 0.00625 | 89.13448 |

As illustrated by the data described in Table 8 (above), the buffered peels containing pyruvic acid (R2 Peels) and sodium pyruvate (R3 Peels) demonstrated similar efficacy.

## Claims

1. A personal care composition comprising:
a blend of fatty acids with less than six carbon atoms; and
a cosmetically acceptable carrier,
wherein the blend of fatty acids comprises:
a first fatty acid selected from: lactic acid; a salt thereof; and a combination thereof; and
a second fatty acid that is selected from pyruvic acid, a salt thereof, and a combination thereof;
wherein the weight ratio of the first fatty acid to the second fatty acid is 4 : 1;
and
wherein the personal care composition is free of acetic acid.

2. The personal care composition according to claim 1, wherein the cosmetically acceptable carrier comprises an ingredient selected from: a surfactant; a particle; a thickening agent; and a combination of two or more thereof.

3. The personal care composition according to any foregoing claim, wherein the composition is in a form selected from a body wash; a hand soap; a shower gel; a wipe; a lotion; a cream; an ointment; an antiperspirant; a peel, and a deodorant.

4. The personal care composition according to claim 3, wherein the antiperspirant and deodorant are in a form selected from: an aerosol; a roll-on; and a stick.

5. The personal care composition according to any foregoing claim, wherein the second fatty acid comprises sodium pyruvate.

6. Use of a personal care composition according to any foregoing claim for increasing the firmness and/or elasticity of the skin of a patient in need thereof by administering to the skin of said patient, an effective amount of a personal care composition according to any one of claims 1 to 5.

7. A personal care composition according to any one of claims 1 to 5 for use in a method for inhibiting:
tyrosinase;
collagenase; and/or
elastase;
in a patient in need thereof, comprising administering to the skin of said patient, an effective amount of the personal care composition.

## Patentansprüche

1. Körperpflegezusammensetzung, umfassend:
eine Mischung aus Fettsäuren mit weniger als sechs Kohlenstoffatomen; und
einen kosmetisch akzeptablen Träger,
wobei die Mischung aus Fettsäuren Folgendes umfasst:
eine erste Fettsäure, ausgewählt aus: Milchsäure; einem Salz davon; und einer Kombination davon; und
eine zweite Fettsäure, die ausgewählt ist aus Brenztraubensäure, einem Salz davon und einer Kombination davon;
wobei das Gewichtsverhältnis der ersten Fettsäure zu der zweiten Fettsäure 4 : 1 beträgt; und
wobei die Körperpflegezusammensetzung frei von Essigsäure ist.

2. Körperpflegezusammensetzung nach Anspruch 1, wobei der kosmetisch akzeptable Träger einen Inhaltsstoff aufweist, der ausgewählt ist aus: einem Tensid; einem Partikel; einem Verdickungsmittel; und einer Kombination aus zwei oder mehr davon.

3. Körperpflegezusammensetzung nach einem der vorstehenden Ansprüche, wobei die Zusammensetzung in einer Form vorliegt, die ausgewählt ist aus einer Körperwaschzusammensetzung; einer Handseife; einem Duschgel; einem Reinigungstuch; einer Lotion; einer Creme; einer Salbe; einem Antitranspirationsmittel; einem Peeling und einem Deodorant.

4. Körperpflegezusammensetzung nach Anspruch 3, wobei das Antitranspirationsmittel und das Deodorant in einer Form vorliegen, die ausgewählt ist aus: einem Aerosol; einem Roll-on; und einem Stift.

5. Körperpflegezusammensetzung nach einem der vorstehenden Ansprüche, wobei die zweite Fettsäure Natriumpyruvat umfasst.

6. Verwendung einer Körperpflegezusammensetzung nach einem der vorstehenden Ansprüche zum Erhöhen der Festigkeit und/oder Elastizität der Haut eines Patienten, der dessen bedarf, durch Auftragen einer wirksamen Menge einer Körperpflegezusammensetzung nach einem der Ansprüche 1 bis 5 auf die Haut des Patienten.

7. Körperpflegezusammensetzung nach einem der Ansprüche 1 bis 5 zur Verwendung in einem Verfahren zum Hemmen von:
Tyrosinase;
Kollagenase; und/oder
Elastase;
bei einem Patienten, der dessen bedarf, umfassend das Auftragen einer wirksamen Menge der Körperpflegezusammensetzung auf die Haut des Patienten.

## Revendications

1. Composition de soins personnels comprenant :
un mélange d'acides gras ayant moins de six atomes de carbone ; et
un support cosmétiquement acceptable,
dans laquelle le mélange d'acides gras comprend :
un premier acide gras choisi parmi : l'acide lactique ; un sel de ce dernier ; et une combinaison de ceux-ci ; et
un deuxième acide gras qui est choisi parmi l'acide pyruvique, un sel de ce dernier et une combinaison de ceux-ci ;
dans laquelle le rapport pondéral du premier acide gras au deuxième acide gras est de 4:1 ; et
dans laquelle la composition de soins personnels est exempte d'acide acétique.

2. Composition de soins personnels selon la revendication 1, dans laquelle le support cosmétiquement acceptable comprend un ingrédient choisi parmi : un tensioactif ; une particule ; un agent épaississant ; et une combinaison de deux ou plus de ceux-ci.

3. Composition de soins personnels selon l'une quelconque des revendications précédentes, dans laquelle la composition est sous une forme choisie parmi un nettoyant pour le corps ; un savon pour les mains ; un gel douche ; une lingette ; une lotion ; une crème ; une pommade ; un anti-transpirant ; un peeling et un déodorant.

4. Composition de soins personnels selon la revendication 3, dans laquelle l'anti-transpirant et le déodorant sont sous une forme choisie parmi : un aérosol ; un roll-on ; et un bâton.

5. Composition de soins personnels selon l'une quelconque des revendications précédentes, dans laquelle le deuxième acide gras comprend du pyruvate de sodium.

6. Utilisation d'une composition de soins personnels selon l'une quelconque des revendications précédentes pour augmenter la fermeté et/ou l'élasticité de la peau d'un patient en ayant besoin par administration à la peau dudit patient, d'une quantité efficace d'une composition de soin personnel selon l'une quelconque des revendications 1 à 5.

7. Composition de soins personnels selon l'une quelconque des revendications 1 à 5, pour une utilisation dans un procédé pour inhiber :
la tyrosinase ;
la collagénase ; et/ou
l'élastase ;
chez un patient en ayant besoin, comprenant l'administration à la peau dudit patient, d'une quantité efficace de la composition de soins personnels.
